# EUROPEAN PATENT SPECIFICATION

(11) **EP 3 325 939 B1**
(45) Date of publication and mention of the grant of the patent: **18.08.2021**
(21) Application number: 16753977.4
(22) Date of filing: 18.07.2016
(51) Int. Cl.: G01N 1/14, G01N 1/40, G01N 33/00, B01D 11/00

(54) **METHOD FOR THE EXTRACTION OF SAP FROM PLANT MATERIAL AND APPARATUS FOR CARRYING OUT THE METHOD**
VERFAHREN ZUR EXTRAKTION VON SAFT AUS PFLANZENMATERIAL UND VORRICHTUNG ZUR AUSFÜHRUNG DES VERFAHRENS
PROCÉDÉ D'EXTRACTION DE LA SÈVE D'UNE MATIÈRE VÉGÉTALE ET APPAREIL POUR EXÉCUTER LE PROCÉDÉ

(30) Priority: 24.07.2015 IT UB20152481
(43) Date of publication of application: 30.05.2018
(73) Proprietor: Avantech Group S.r.l., 84012 Angri (SA) (IT)
(72) Inventor: YASEEN, Thaer, 70010 Locorotondo BA (IT)
(74) Representative: Perani & Partners S.p.A.
(86) International application number: PCT/IB2016/054256
(87) International publication number: WO 2017/017555

(56) References cited:
- WO-A1-2005/057206
- WO-A1-2012/035117
- CN-A- 103 759 995
- US-A1- 2014 193 303

## Description

The present invention refers to a method for extracting the sap from a sample of plant material to carry out the diagnosis of organisms present therein through molecular and serological analysis.

In the present patent application, the term sample of plant material is meant to indicate an amount of living tissue of a plant, particularly a piece of a branch.

The description also refers to an apparatus for carrying out the method.

In accordance with the prior art, the methods for extracting sap comprise a step of shredding the plant tissue, sometimes also in the presence of liquid nitrogen, with the addition of various chemical substances, often harmful, in order to be able to purify the nucleic acids or the proteins present in the plant.

The known and conventional methods for extracting the nucleic acids or the proteins from plant material thus need to be carried out in a laboratory and take a long time to be performed as well as involving the use of very expensive materials or examination kits.

In cases of epidemics spread over a large territory, like, for example, that of the Mediterranean olive tree caused by the bacterium *Xylella fastidiosa*, since it is necessary in a short space of time to gain possession of sap samples, sufficient to carry out the analyses, collected in area distributed over the territory even very far apart, the aforementioned known conventional methods for collecting and extracting sap do not have suitable application.

The patent application WO 2012/035117 A1 discloses a method for the extraction of components from a plant material comprising:
- preparing a sample of plant material having a first and a second end having each a predetermined cross section,
- injecting a liquid through its first end, and
- collecting, at the second end of the sample, the liquid exiting therefrom.

The purpose of the present invention is to have a method for collecting and extracting the sap from a sample of plant material that can also be used directly in the field, in a short time period, overcoming the aforementioned drawbacks in relation to conventional known methods.

A further purpose is to have an easily transportable apparatus for carrying out the method also in the field directly at the plant from which the plant sample from which to extract the sap to be examined is taken.

Such purposes are achieved by a method according to claim 1.

Again in accordance with the invention, the liquid to be injected into the sample of plant material and to be collected together with the sap comprises a saline buffer.

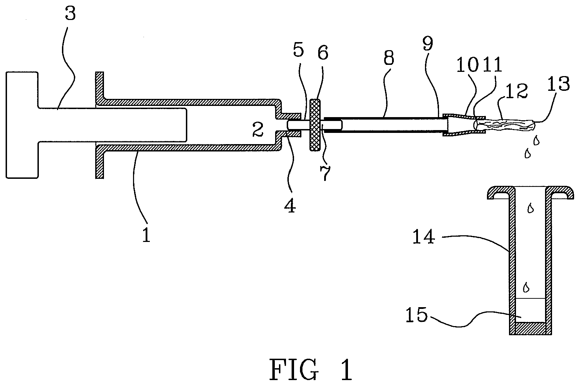

The invention will now be described more with reference to a preferred apparatus for carrying out the method, illustrated as an indicating example and not for limiting purposes, in figure 1 of the attached drawing.

With reference to the aforementioned figure 1, the apparatus comprises a pumping device 1, preferably in the form of a syringe, in the cavity 2 of which a dose of saline buffer is loaded that, through the piston 3, actuated manually and therefore at low pressure, pushes the saline buffer through the outlet 4 of the syringe 1.

The latter is connected to the inlet fitting 5 of a filter element 6.

Preferably, the filter element 6 is provided with a filter of at least 0.22 µm in order to prevent the possible presence of fungi or bacteria in the sap of the sample of plant material being able to migrate and pollute the saline buffer present in the syringe 1 preventing its use for the extraction of the sap from another different sample of plant material.

The outlet fitting 7 of the filter element 6 is connected to a tubular duct 8 which is preferably in the form of a flexible hose, for example with a diameter of 5 mm and equipped with a length equal to about 2-3 cm.

Between the end 9 of the duct 8 and the first end 11 of the sample of plant material 12 there is a joining element 10 to carry out both the sealed connection and to adapt the section of the duct 8 to the cross section of the sample of plant material 12 from which it is wished to extract the sap.

At the second end 13 of the sample of plant material, opposite the end 11, through a conventional test tube, indicated with 14, the liquid is collected, schematically indicated with 15, which exits from the sample of plant material. Such a liquid comprises both the saline buffer injected and the sap that it carries with it crossing the sample of plant material 12.

In accordance with the invention, the sample of plant material 12 consists of a segment, taken from a branch of the plant, with average age of between one and two years.

Preferably, the branch segment has a length of between 4 and 5 cm and a cross section with diameter of between 3 and 5 mm.

From what has been described above, it can be appreciated that the method for extracting the sap according to the invention is carried out by injecting the sample of plant material 12 using the syringe 1 axially, with a saline buffer and collecting, in the test tube 14, at the end 13, an amount of about 100-200 µl of liquid consisting of saline buffer and sap.

The operation is carried out in the field in a very short time, of the order of 30-60 seconds, making the method highly productive particularly when numerous samples of sap must be taken quickly and at locations far apart from one another.

The liquid, thus extracted, does not need preparation operations and can be subjected directly to the conventional serological analyses, such as ELISA (Enzyme Linked Immuno Sorbent Assay) or DTBIA (Direct Tissue Blot Immuno Assay) widely used in the diagnosis of plant organisms of different kinds such as viruses, bacteria and fungi.

Only in the case in which the liquid collected in the test tube 14 needs to be subjected to molecular analysis techniques, like for example LAMP (Loop-mediated isothermal AMPlification) or PCR (Polymerase Chain Reaction), is it necessary to subject it to the conventional prior heating to 95°C for at least 10 minutes in order to extract and denature the nucleic acids of the organisms present in the plant tissue under examination.

The invention, as illustrated and described above, can undergo variants, particularly in terms of shape and size relative to the sample of plant material from which to extract the sap, all in any case covered by the scope of protection of the invention as claimed below.

## Claims

1. Method for the extraction of sap from plant material comprising:
- preparing a sample (12) of plant material having a first (11) and a second (13) end, the ends being longitudinally spaced from each other and each having a predetermined cross section, the sample being a segment taken from a branch of a plant with an average age of between one and two years,
- injecting the sample, through its first end (11), with a predetermined amount of extraction liquid and
- collecting, at the second end (13) of the sample, the liquid exiting therefrom.

2. Method according to claim 1 wherein said branch segment has a length of at least 4 cm and a cross section with diameter of at least 3 mm.

3. Method according to any one of claims from 1 to 2 wherein said extraction liquid comprises a saline buffer.

4. Method according to any one of claims from 1 to 3 including a step in which the extraction liquid, before its injection into said sample of plant material, is made to pass through a filter element.

## Patentansprüche

1. Verfahren zur Extraktion von Saft aus Pflanzenmaterial, umfassend:
- Vorbereiten einer Probe (12) von Pflanzenmaterial aufweisend ein erstes (11) und ein zweites (13) Ende, wobei die Enden in Längsrichtung voneinander beabstandet sind und jeweils einen vorbestimmten Querschnitt aufweisen; die Probe ein Segment ist, das aus einem Zweig einer Pflanze mit einem Durchschnittsalter zwischen einem und zwei Jahren entnommen wurde,
- Injizieren der Probe durch ihr erstes Ende (11) mit einer vorbestimmten Menge an Extraktionsflüssigkeit und
- Sammeln, am zweiten Ende (13) der Probe, der daraus austretenden Flüssigkeit.

2. Verfahren nach Anspruch 1 wobei das Zweigsegment eine Länge von mindestens 4 cm und einen Querschnitt mit einem Durchmesser von mindestens 3 mm aufweist.

3. Verfahren nach irgendeinem der Ansprüche 1 bis 2, wobei die Extraktionsflüssigkeit einen Salzpuffer umfasst

4. Verfahren nach irgendeinem der Ansprüche von 1 bis 3, einschließlich eines Schritts, in dem die Extraktionsflüssigkeit, vor ihrer Injektion in die Probe des Pflanzenmaterials, durch ein Filterelement geleitet wird.

## Revendications

1. Procédé pour l'extraction de sève d'un matériau végétal comprenant :
- préparer un échantillon (12) de matériau végétal ayant une première (11) et une deuxième (13) extrémité, les extrémités étant espacées longitudinalement l'une de l'autre et ayant chacune une section transversale prédéterminée, l'échantillon étant un segment pris à partir d'une branche d'une plante avec un âge moyen entre un et deux ans,
- injecter dans l'échantillon, à travers sa première extrémité (11), une quantité prédéterminée de liquide d'extraction et
- récolter, au niveau de la deuxième extrémité (13) de l'échantillon, le liquide sortant de celle-ci.

2. Procédé selon la revendication 1 dans lequel ledit segment de branche a une longueur d'au moins 4 cm et une section transversale avec un diamètre d'au moins 3 mm.

3. Procédé selon l'une quelconque des revendications de 1 à 2 dans lequel ledit liquide d'extraction comprend une solution tampon saline.

4. Procédé selon l'une quelconque des revendications de 1 à 3 incluant une étape dans laquelle le liquide d'extraction, avant son injection dans ledit échantillon de matériau végétal, est fait passer à travers un élément filtrant.
